# EUROPEAN PATENT APPLICATION

(11) **EP 2 266 966 A1**
(43) Date of publication of application: **29.12.2010**
(21) Application number: 09382093.4
(22) Date of filing: 11.06.2009
(51) Int. Cl.: C07D 277/32

(54) **A process for the preparation of febuxostat**

(71) Applicant: Chemo Ibérica, S.A., 08028 Barcelona (ES)
(72) Inventor: Castaldi, Graziano, 28072 Briona (NO) (IT); Rasparini, Marcello, 27010 Cura Carpignano (PV) (IT); Sillani, Laura, 28014 Maggiora (NO) (IT)
(74) Representative: ZBM Patents

(57) **Abstract**

The present invention relates to a process for the preparation of Febuxostat, useful in the pharmaceutical field for the treatment of the hyperuricemia.

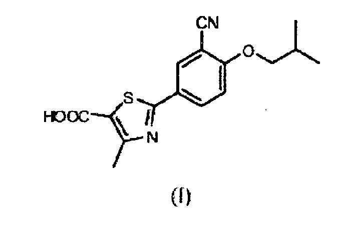

## Description

### Field of the invention

The present invention relates to a process for the preparation of Febuxostat, useful in the pharmaceutical field for the treatment of the hyperuricemia.

### Background of the invention

The hyperuricemia (elevated levels of uric acid in the blood) causes gout, wherein crystals of uric acid are deposited on the articular cartilage of joints, tendons and surrounding tissues. Gout is marked by painful attacks of acute arthritis initiated by the crystallisation of urates within and about the joints. Besides, hyperuricemia causes renal insufficiency; it is considered to be a factor for coronary disease and it is suggested to closely relate to development of diseases of adult people, such as hypertension. Therefore, treatment of the hyperuricemia can be effective not only for treating gout, but also for preventing various diseases relating to daily nutrition and developing in the course of advancement of age. The principal medications used to treat hyperuricemia act by decreasing the blood concentration of uric acid. These are xanthine oxidase inhibitors, such as Allopurinol, which reduce the production of uric acid; and uricosurics, which reduce the reabsorption of uric acid excreted by the kidneys.

Allopurinol, a structural isomer of hypoxanthine, inhibits xanthine oxidase and prevents the formation of uric acid from hypoxanthine and xanthine, to thus lower the serum uric acid level. It has long been used to treat hyperuricemia, but its serious side effects, such as skin rash, hepatitis, worsened renal function, myelogenetic troubles have led to the development of other xanthine oxidase inhibitors, such as Febuxostat.

Febuxostat is a compound of formula (I) 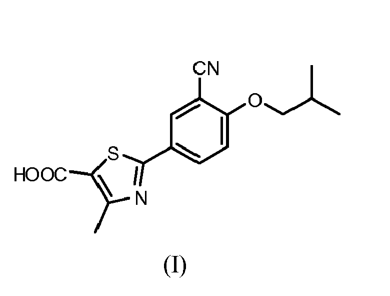 chemically known as 2-(3-cyano-4-isobutoxyphenyl)-4-methylthiazole-5-carboxylic acid, which is disclosed in EP 513379 B1 and marketed under the trade name Adenuric^{®}.

The synthesis of Febuxostat was disclosed in EP 513379 B1, wherein the process involves the condensation of 4-hydroxy-3-nitrobenzaldehyde with hydroxylamine hydrochloride in the presence of sodium formate to give 4-hydroxy-3-nitrobenzonitrile. Conversion of the cyano group into a thioamide and subsequent reaction with ethyl 2-chloroacetoacetate provide ethyl 2-(4-hydroxy-3-nitrophenyl)-4-methyl-5-thiazolecarboxylate. Alkylation of the 4-hydroxyl group with isopropyl bromide, and catalytic hydrogenation of the 3-nitro moiety provides ethyl 2-(3-amino-4-isobutoxyphenyl)-4-methyl-5-thiazolecarboxylate. Finally, reaction of the latter with sodium nitrite and subsequent hydrolysis of the ethyl carboxylate give Febuxostat.

The active substances prepared by the processes known until now can be obtained after running through a large number of process steps. The main drawbacks associated with the known syntheses are the harsh conditions, the toxic reagents to form the thioamide and the use of highly toxic cyanides.

There is therefore the need for an alternative synthesis for the industrial preparation of Febuxostat, which makes use of commercially available, cheaper, and non-toxic reagents. An object of the present invention is to provide an alternative, efficient, economic and commercially useful process for the manufacture of Febuxostat, that avoids the above-identified problems.

### Summary of the invention

It has now been found an economic process for the preparation of Febuxostat in a straightforward manner.

In one embodiment, the method of the present invention involves a process for preparing Febuxostat of formula (I), or a pharmaceutically acceptable salt, polymorph, hydrate or solvate thereof, 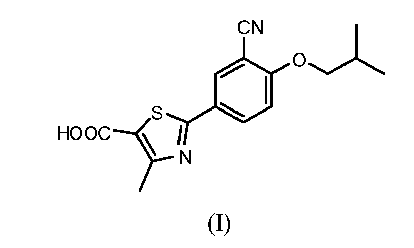 which comprises:
a) reacting a compound of formula (III) 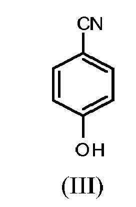 with a brominating agent, optionally in the presence of an acidic catalyst, to give a compound of formula (IV) 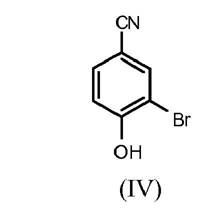
b) alkylating a compound of formula (IV) with a compound R₂-X₁, wherein:
   R₂ is isobutyl,
   X₁ is a leaving group, in the presence of a base, to give a compound of formula (V) 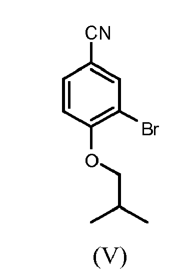
c) converting the cyano compound of formula (V), into the compound of formula (VI), 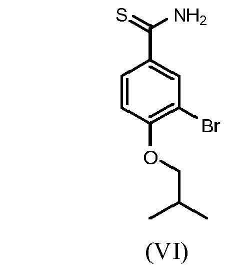 by reaction with a mixture of sodium hydrosulphite, or a hydrate thereof, and magnesium chloride, or a hydrate thereof, in a solvent;
d) cyclising a compound of formula (VI) with a compound of formula (VII) 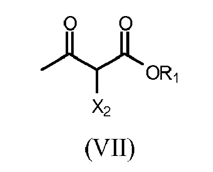 wherein:
   R₁ is hydrogen, or a C₁-C₆ alkyl group optionally substituted; and
   X₂ is a leaving group,
      to give a compound of formula (VIII) 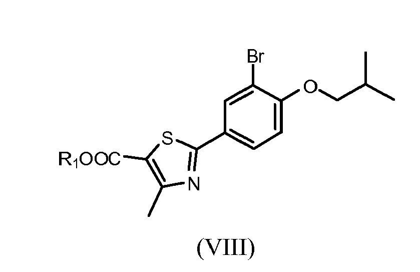 wherein R₁ is as defined above;
e) cyanating a compound of formula (VIII), as defined above, with potassium ferrocyanide, in the presence of a basic agent, and a catalytic system, comprising a metal and optionally an organic ligand; to give the compound of formula (II) 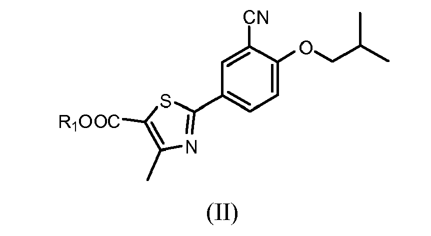 wherein R₁ is as defined above;
f) optionally hydrolysing the compound of formula (II), wherein R₁ is C₁-C₆ alkyl group optionally substituted.

The process of the present invention offers several advantages over previous methods of making Febuxostat, and is very useful for the industrial preparation of Febuxostat. The present method is conducted under very mild conditions and makes use of generally non toxic or low toxic reagents, like sodium hydrosulphite, magnesium chloride and potassium ferrocyanide.

### Detailed description of the invention

All terms as used herein in this application, unless otherwise stated, shall be understood in their ordinary meaning as known in the art. Other more specific definitions for certain terms as used in the present application are as set forth below and are intended to apply uniformly through-out the specification and claims unless an otherwise expressly set out definition provides a broader definition.

The terms "alkyl", "C₁-C₆ alkyl" refer to a straight or branched hydrocarbon having from 1 to 6 carbon atoms. Examples of alkyl groups include, without limitation, methyl, ethyl, *n-*propyl, isopropyl, *n*-butyl, *sec*-butyl, isobutyl, *tert*-butyl, *n*-, *sec*-, *iso*-, *tert-,* and *neo*-pentyl, *n*-, *sec*-, *iso*-, *tert*-, and *neo*-hexyl, and the like. A preferred C₁-C₆ alkyl group of the present invention is ethyl.

The term "halogen" refers to chlorine, bromine, fluoride or iodine.

The term "substituted" refers to groups substituted with from 1 to 5 substituents selected from the group consisting of C₁-C₈ alkyl, halogen, aryl, and the like.

The term "C₁-C₈ alkyl" refers to a straight or branched hydrocarbon having from 1 to 8 carbon atoms, hence comprehensive of the aforementioned "C₁-C₆ alkyl" and also comprising *n*-, *sec*-, *iso*-, *tert*-, and *neo*-heptyl and *n*-, *sec*-, *iso*-, *tert*-, and *neo*-octyl, and the like.

The term "aryl" refers to any aromatic carbocyclic ring system of 1 or 2 ring moieties, either fused or linked to each other through a single bond. Suitable aryl groups include, but are not limited to, phenyl, α- or β- naphthyl, biphenyl, indanyl, indenyl, and the like.

The term "leaving group" has the same meaning to the skilled man (Advanced Organic Chemistry: reactions, mechanisms and structure - Third Edition by Jerry March, John Wiley and Sons Ed.; 1985 page 179) and represents a group which is part of and attached to a substrate molecule; in a reaction where the substrate molecule undergoes a displacement reaction (with for example a nucleophile), the leaving group is then displaced. Preferred leaving groups are halogen, such as chloride, bromide, iodide; sulfonic esters, such as mesylate, tosylate, triflate; more preferably bromide or chloride.

The term "pharmaceutically acceptable salts" refers to the relatively non-toxic base-addition salts of the compounds of the present invention. These salts may be prepared in situ during the final isolation and purification of the compounds. In particular, the basic-addition salts may be prepared by separately reacting the compound with an organic or inorganic base and isolating the salt thus formed. The resulting salts are, for example, metal salts, particularly alkali metal salts, alkaline-earth metal salts and transition metal salts (such as sodium, potassium, calcium, magnesium, aluminium, zinc), or salts obtained with bases, such as ammonia or secondary or tertiary amines (such as diethylamine, triethylamine, piperidine, piperazine, morpholine), or with basic amino-acids, or with osamines (such as meglumine), or with aminoalcohols (such as choline, diethanolamine, 3-aminobutanol and 2-aminoethanol).

The term "hydrate" refers to a solvate comprising a disclosed or claimed compound and a stoichiometric or non-stoichiometric amount of water.

The term "solvate" refers to a molecular complex comprising a disclosed or claimed compound and a stoichiometric or non-stoichiometric amount of one or more solvent molecules (*e.g.*, ethanol).

Generally, the chemical transformations described throughout the specification may be carried out using substantially stoichiometric amounts of reactants, though certain reactions may benefit from using an excess of one or more of the reactants. Additionally, many of the reactions disclosed throughout the specification, may be carried out at room temperature, but particular reactions may require the use of higher or lower temperatures, depending on reaction kinetics, yields, and the like. Furthermore, many of the chemical transformations may employ one or more compatible solvents, which may influence the reaction rate and yield. Depending on the nature of the reactants, the one or more solvents may be polar protic solvents, polar aprotic solvents, non-polar solvents, or some combination.

The compounds obtained by the chemical transformations of the present invention, can be used for the following steps without further purification or can be effectively separated and purified by crystallization, or by transforming then into a salt or by washing with an organic solvent or with an aqueous solution, eventually adjusting pH.

Any suitable solvent known to those skilled in the art can be used, for the crystallization or for washing. Suitable solvents, for instance, are non polar solvent, such as hexane, toluene; polar solvent, for example, acetonitrile, dioxane, ethanol, N,N-dimethylacetamide, dimethylformamide. The solvent may be selected between an ether, such as diethyl ether, tetrahydrofuran or dioxane; a chlorinated hydrocarbon, such as dichloromethane or chloroform; an ester, such as ethyl acetate; an hydrocarbon, such as toluene, hexane; an alcohol, such as methanol, ethanol, isopropanol; a ketone, such as acetone; etc.

The process of the invention for preparing Febuxostat of formula (I) is illustrated in Scheme 1.

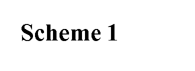

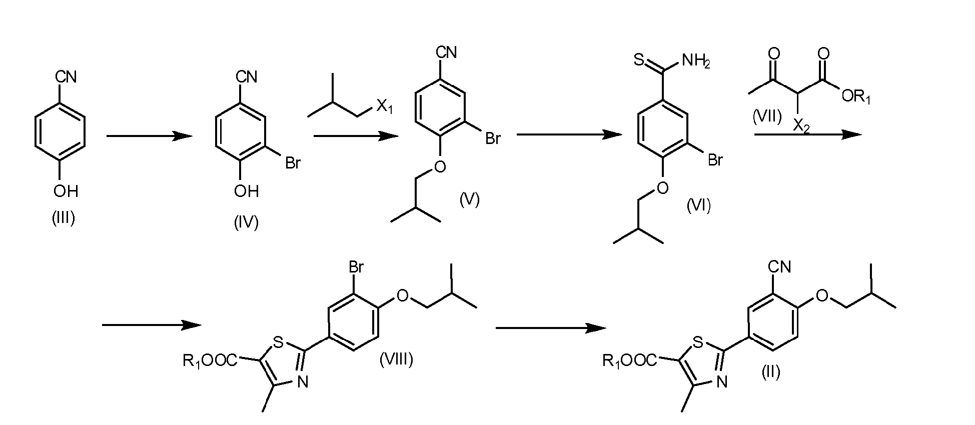

wherein R₁, X₁ and X₂ are as defined above.

Selective bromination in *ortho*-position of the commercially available 4-cyanophenol of formula (III) 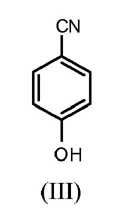 optionally in the presence of an acidic catalyst, according to a procedure described in literature (Oberhauser, T. J. Org. Chem. 1997, 62, 4504), gives a compound of formula (IV). Any suitable brominating reagent known to those skilled in the art can be used. Examples of a suitable brominating reagent include, but are not limited to, bromine, tetraalkylammonium tribromide, dioxane dibromide or N-bromosuccinimide (NBS), preferably NBS. The bromination can occur in a polar solvent, such as dimethylformamide, N,N-dimethylacetamide, propylene carbonate, acetonitrile, preferably acetonitrile, optionally in the presence of an acidic catalyst, such as aqueous sulphuric acid, paratoluensulphonic acid, fluorosulphuric acid, fluoroboric acid etherate, trifluoromethanesulphonic acid, preferably trifluoromethanesulphonic acid. The phenol/acidic catalyst ratio is advantageously comprised from 1.0 to 1.5, preferably from 1.0 to 1.2.

The compound of formula (V) is obtained by alkylation of a compound of formula (IV) with R₂-X₁, wherein R₂ is isobutyl and X₁ is a leaving group, preferably chloride or bromide, more preferably bromide; in the presence of a basic agent, in a suitable solvent. Any suitable solvent known to those skilled in the art can be used, for instance a polar solvent, for example N,N-dimethylacetamide, dimethylformamide. Any suitable basic agent known to those skilled in the art can be used, for instance an organic base, for example straight or branched tertiary amines, such as triethylamine, diisopropyl ethyl amine, tetramethylethylenediamine, tributylamine, pyridine, N-methylmorpholine, tetramethylurea, methylpyrrolidinone, 4-dimethylaminopyridine, proton sponge or dimethylaniline; or an inorganic base, for example alkali or alkali earth hydroxides, such as sodium hydroxide, calcium hydroxide, or alkali or alkaline-earth metal carbonates, such as sodium or potassium carbonate. Preferably the base is an inorganic base, more preferably potassium carbonate. The preferred base is employed in a molar ratio comprised between 1.0 and 2.0 to the compound (IV), preferably between 1.0 and 1.5.

The alkylating reagent is employed in a molar ratio comprised between 1.0 and 2.0 to the compound (IV), preferably in a molar ratio between 1.0 and 1.5.

The alkylation reaction is carried out at a suitable temperature. A suitable temperature is a temperature in order for the reaction to be complete. The temperature may range from room temperature to the reflux temperature, preferably the temperature is between 60 and 100°C. The conversion of the nitrile of formula (V) into the thioamide of formula (VI) is carried out with a very mild process, by using non toxic reagents. Said reagents are a mixture of sodium hydrosulphite, in a hydrate form or not, and magnesium chloride, or a hydrate thereof, such as hexahydrate, in a suitable solvent, such as polar aprotic solvents, for example, N-methyl pyrrolidone, dimethylsulfoxide, dimethylformamide, dimethylacetamide, preferably dimethylformamide.

The molar ratio of sodium hydrosulphite with respect to substrate may be comprised from 1 to 5, preferably from 1 to 3.

The magnesium chloride/sodium hydrosulphite ratio is advantageously comprised from 1:1 to 1:5, preferably from 1:1 to 1:2.

The reaction may be carried out at room temperature. The thioamide of formula (VI) is obtained in high yield and may be purified by crystallization.

The cyclisation of the thioamide of formula (VI), with a compound of formula (VII), 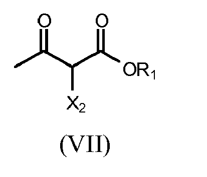 wherein:
R₁ is hydrogen, or a C₁-C₆ alkyl group optionally substituted;
X₂ is a leaving group,
   gave the derivative of formula (VIII).

Preferably R₁ is ethyl, and X₂ is halogen, more preferably chlorine. In a preferred embodiment, the compound of formula (VII) is ethyl 2-chloroacetoacetate.

According to another preferred embodiment, the reaction is carried out in a polar solvent, such as alcohols, for example methanol, ethanol, *iso*-propanol, *n*-propanol; dimethylformamide, acetonitrile. More preferably the solvent is ethanol.

The cyclization is carried out at a suitable temperature, comprised from room temperature to the reflux temperature, preferably from 50 to 80°C.

The cyanation of the aryl bromide of formula (VIII), wherein R₁ is hydrogen, or a C₁-C₆ alkyl group optionally substituted, preferably ethyl, with the low toxic potassium ferrocyanide trihydrate, in the presence of a basic agent and a catalyst gave the compound of formula (II). Any suitable base known to those skilled in the art can be used. Examples of suitable bases include, but are not limited to, inorganic bases, such as alkali or alkali earth hydroxides, for instance sodium hydroxide, calcium hydroxide; or alkali or alkaline-earth metal carbonates, such as sodium carbonate. A preferred basic agent is sodium carbonate.

Any suitable catalyst known to those skilled in the art can be used. The reaction is preferably catalysed by a catalytic system comprising a metal and optionally an organic ligand. Preferably, the catalyst comprises a transition metal selected from Pd or Cu, more preferably Pd. Preferred catalyst are Pd(OAc)₂ or Pd/C.

The amount of catalyst to be employed can vary within wide limits. In general, an amount of between 0.1% moles and 5% moles with respect to substrate gives satisfactory results. Preferably, the concentration of a metal is between 1% molar and 2% molar with respect to substrate.

When an organic ligand is used, the components of the catalytic system may be added separately to the reaction mixture. The organic ligand complexes the metal and forms the active metal-ligand complex catalyst. The organic ligand may be selected from the group consisting of a phosphine, such as triarylphosphine, preferably P(Ph)₃; trialkylphosphine; a bidendate diphosphine ligand, comprising xanthene-based diphosphines (Xantphos ligands), such as 9,9-dimethyl-4,5-bis(diphenylphosphino)xanthene (Xantphos), 2,7-di-tert-butyl-9,9-dimethyl-4,5-bis(diphenylphosphino)xanthene (tBu-Xantphos); phenoxazine-based diphosphines (Nixantphos ligands), such as 4,6-bis(diphenylphosphino)phenoxazine; (oxydi-2,1-phenylene)bis(diphenylphosphine) (DPEphos); 1,1'-bis(diphenylphosphino)ferrocene (dppf); or a bidentate amine ligand, such as ethylenediamine, o-phenylene diamine, tetramethylethylenediamine, propane-1,3 diamine, preferably ethylenediamine.

When in the compound of formula (II), R₁ is hydrogen, Febuxostat is obtained. When in the compound of formula (II), R₁ is a C₁-C₆ alkyl group optionally substituted, the compound of formula (II) can be optionally hydrolysed in conditions well known to those skilled in the art, for instance in the presence of a basic or an acidic agent, to provide Febuxostat. Many of the compounds described in this disclosure, are capable of forming pharmaceutically acceptable salts. These salts include, without limitation, base salts. Salts which are not pharmaceutically acceptable may still be valuable as intermediates.

One may prepare an acceptable base salt by contacting a compound's free acid or zwitterion with a sufficient amount of a desired base to produce a salt. If the salt precipitates from solution, it may be isolated by filtration; otherwise, the salt may be recovered by evaporating the solvent.

One may also regenerate the free acid by contacting the base salt with an acid. Though certain physical properties of the free acid and its respective base salt may differ (e.g., solubility, crystal structure, hygroscopicity, etc. ), a compound's free acid and base salt are otherwise the same for purposes of this disclosure.

As mentioned above the compounds of the invention have useful therapeutic properties. It is contemplated that the compounds of the present method can be found or isolated in the form of hydrates or solvates, in different polymorphic forms, i.e., different crystalline forms, which are considered to fall within the scope of the present invention. The compounds of the invention may be administered per se or, preferably, as a pharmaceutical composition.

While the present invention has been described in terms of its specific embodiments, certain modifications and equivalents will be apparent to those skilled in the art and are included within the scope of the present invention.

In the following, the present invention shall be illustrated by means of some examples, which are not construed to be viewed as limiting the scope of the invention.

### Examples

The following abbreviations refer respectively to the definitions below:
CF₃SO₃H (Trifluoromethanesulfonic acid); DMA (N,N-dimethylacetamide); EtOAc (Ethyl acetate); EtOH (Ethanol); GC (Gas Chromatography); KF (Karl Fisher); K₂CO₃ (Potassium carbonate); KI (Potassium iodide); MgCl₂ (Magnesium chloride); MTBE (Methyl *tert*-butyl ether); NBS (N-Bromosuccinimide); NaSH (Sodium hydrosulfite); Na₂CO₃ (Sodium carbonate); NMP (N-methyl pyrrolidone); Pd(OAc)₂ (Palladium acetate); TLC (Thin Layer Chromatography).

GC Agilent 6850 Gas-Chromatograph.

HP1 Column, length 30 m, 0.32 mm I.D., 0.25 µm film thickness.

DRIFT was performed on a Perkin Elmer instrument.

### Example 1

### 3-Bromo-4-hydroxybenzonitrile (IV)

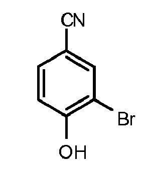

4-Cyanophenol (III) (5.0 g, 0.042 mol) was dissolved in acetonitrile (50 mL), under a nitrogen atmosphere. The mixture was cooled to -15°C, and CF₃SO₃H was added (3.7 mL, 6.3 g, 0.042 mol). The temperature was kept under 10°C and NBS (8.2 g, 0.046 mol) was added in 6 portions. The reaction mixture was brought at room temperature and was stirred for 4 hours under a nitrogen atmosphere, monitoring by TLC (Hexane/EtOAc 75:25, UV, KMnO₄). When the starting material was completely consumed, the mixture was diluted with an aqueous solution of Na₂CO₃ and extracted with MTBE (3 × 50 mL). The combined organic layers were dried over sodium sulphate and the solvent was removed under reduced pressure to afford the title compound, as a white solid (7.7 g, 93%).
¹H NMR (DMSO, 300 MHz, 300 K): δ= 8.04 ppm (d, J=2.2 Hz, 1H), 7.63 (dd, J=2.2 Hz, J=8.5 Hz, 1H), 7.04 (d, J=8.5, 1H).

### Example 2

### 3-Bromo-4-isobutoxybenzonitrile (V)

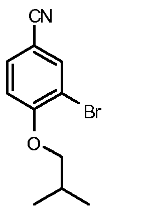

3-Bromo-4-hydroxybenzonitrile (IV) (25.17 g, 0.127 mol) was dissolved in DMA (100 mL), under a nitrogen atmosphere. To the resultant solution, K₂CO₃ (26.0 g, 0.190 mol) was added, followed by *i*-butyl bromide (17.7 mL, 22.30 g, 0.165 mol) and KI (2.0 g, 0.013 mol). The mixture was heated to 80 °C overnight. The reaction was monitored by TLC (Hexan/EtOAc 8:2, UV, KMnO₄) and by GC. If the conversion was still incomplete after 18 hours, an additional aliquot of *i*-butyl bromide and K₂CO₃ were added.

The mixture was then diluted with H₂O (100 mL) and extracted with MTBE (2 x 100 mL). The combined organic layers were washed with water and brine, dried over sodium sulphate and the solvent was removed under reduced pressure to afford the title compound, as a brown oil (26.6 g, 82%).

¹H NMR (CDCl₃, 300 MHz, 300 K): δ = 7.77 ppm (d, *J*=1.8 Hz, 1H), 7.53 (dd, *J*=2. Hz, *J*=8.5 Hz, 1H), 6.87 (d, *J*=8.5 Hz, 1H), 3.81 (d, *J*=6.4 Hz, 2H), 2.19-2.10 (m, 1H), 1.04 (d, *J*=6.7,6H).

### Example 3

### 3-Bromo-4-isobutoxybenzothioamide (VI)

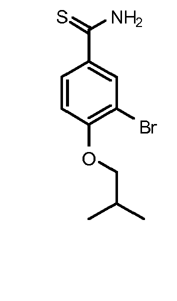

To a suspension of NaSH (hydrate, 70 % w/w) (11.98 g, 0.150 mol) and MgCl₂.6 H₂O (15.20 g, 0.075 mol) in DMF (100 mL), 3-bromo-4-isobutoxybenzonitrile (V) (19.01 g, 0.075 mol) was added. The mixture was stirred at 20 °C for 18 hours, monitoring by TLC (Hexane/EtOAc 7:3, UV, KMnO₄) or GC. When the starting material was completely consumed, the mixture was poured in water (200 mL). The precipitated solid was washed with water and then stirred in IN HCl (200 mL) for 20 minutes. After filtration and washing with water, the product was azeotropically dried with toluene under reduced pressure (rotavapor) and crystallized from toluene and hexane, obtaining the title compound, as a yellow solid (19.80 g, 92% yield).

¹H NMR (CDCl₃, 300 MHz, 300 K): δ = 8.09 ppm (d, *J*=1.8 Hz, 1H), 7.86 (dd, *J*=1.8 Hz, *J*=8.5 Hz, 1H), 6.83 (d, *J*=8.5 Hz, 1H), 3.82 (d, *J*=6.4 Hz, 2H), 2.19-2.13 (m, 1H), 1.05 (d, *J*=6.7 Hz, 6H).

### Example 4

### Ethyl 2-(3-bromo-4-isobutoxyphenyl)-4-methylthiazole-5-carboxylate (VIII)

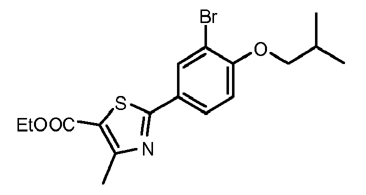

3-Bromo-4-isobutoxybenzothioamide (VI) (19.89 g, 0.069 mol) was dissolved in EtOH (130 mL) and added of ethyl 2-choroacetoacetate (9.5 mL, 11.30 g, 0.069 mol). The mixture was refluxed for 16 hours under nitrogen atmosphere monitoring by TLC (Hexane/EtOAc 8:2, UV). When the starting material was completely consumed, the mixture was cooled to room temperature and the product filtered. The title product was crystallized from ethanol, obtaining a white solid (21.04 g, 76% yield).

¹H NMR (CDCl₃, 300 MHz, 300 K): δ = 8.16 ppm (d, *J*=2.1 Hz, 1H), 7.80 (dd, *J*=2.1 Hz, *J*=8.6 Hz, 1H), 6.87 (d, *J*=8.6 Hz, 1H), 4.33 (q, *J*=7.1 Hz, 2H), 3.82 (d, *J*=6.4 Hz, 2H), 2.74 (s, 3H), 2.19-2.12 (m, 1H), 1.36 (t, *J*=7.1 Hz, 3H), 1.07 (d, *J*=6.7 Hz, 6H).

### Example 5

### Ethyl 2-(3-cyano-4-isobutoxyphenyl)-4-methylthiazole-5-carboxylate (Febuxostat ethyl ester) (II)

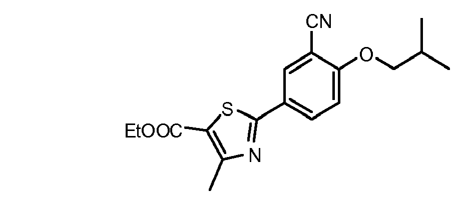

Potassium ferrocyanide trihydrate (0.345 g, 0.82 mol) was dried in a three-necked flask under reduced pressure (5x10⁻² Torr), with magnetic stirring at 80 °C for 3 hours (final KF=1.47%). The flask was cooled to room temperature and then placed under nitrogen atmosphere, fitted with reflux condenser and thermometer and charged under a nitrogen sweep with ethyl 2-(3-bromo-4-isobutoxyphenyl)-4-methylthiazole-5-carboxylate (VIII) (1.66 g, 4.17 mmol), Na₂CO₃ (0.441 g, 4.17 mmol), Pd(OAc)₂ (18 mg, 80 µmol) and dry NMP (18 mL). Three vacuum-nitrogen cycles were performed and the reaction was heated to 145 °C for 48 hours, monitoring by GC until complete disappearance of the starting material. The black mixture was cooled to room temperature, diluted with water (20 mL) and extracted with dichloromethane. The combined organic layers were diluted with toluene and washed with water until complete removal of NMP. After drying over sodium sulphate, the residue was crystallized from ethanol, obtaining the title compound, as a colourless solid.
¹H NMR (CDCl₃, 300 MHz, 300 K) δ = 8.12 ppm (d, *J*=2.2 Hz, 1H), 8.04 (dd, *J*=2.2 Hz, *J*=8.8 Hz, 1H), 6.97 (d, *J*=8.8 Hz, 1H), 4.32 (q, *J*=7.1 Hz, 2H), 3.86 (d, *J*=6.4 Hz, 1H), 2.72 (s, 3H), 2.19-2.12 (m, 1H), 1.35 (t, *J*=7.1 Hz, 3H), 1.06 (d, *J*=6.7 Hz, 6H).
¹³C NMR (CDCl₃, 75 MHz, 300 K) δ=166.0, 161.4, 161.0, 160.0, 131.5, 131.0, 125.0, 120.9,114.3,111.6,102.0,74.7,60.3,27.1,18.0,16.4,13.3
IR (DRIFT) 3120, 3069, 2973, 2933, 2875, 2590, 2226 (CN stretching) 1710, 1607, 1510, 1471, 1429, 1369, 1301, 1264 cm⁻¹

## Claims

1. A process for preparing Febuxostat of formula (I), or a pharmaceutically
acceptable salt, polymorph, hydrate or solvate thereof, 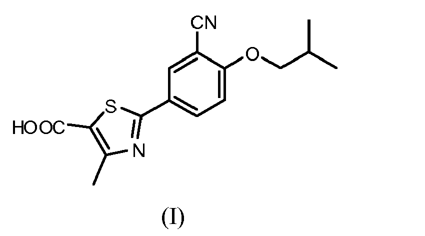 which comprises:
a) reacting a compound of formula (III) 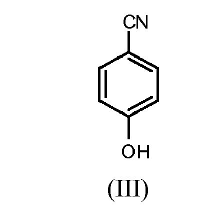 with a brominating agent, optionally in the presence of an acidic catalyst, to give a compound of formula (IV) 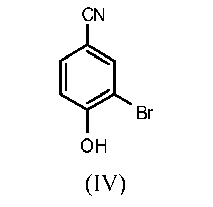
b) alkylating a compound of formula (IV) with a compound R₂-X₁, wherein:
R₂ is isobutyl,
X₁ is a leaving group,
in the presence of a base, to give a compound of formula (V) 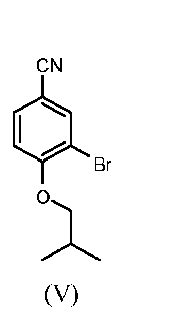
c) converting the cyano compound of formula (V), into the compound of formula (VI), 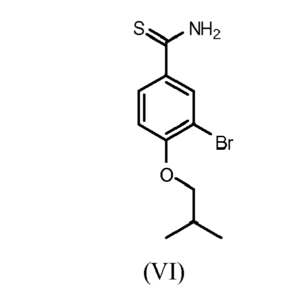 by reaction with a mixture of sodium hydrosulphite, or a hydrate thereof, and magnesium chloride, or a hydrate thereof, in a solvent;
d) cyclizing a compound of formula (VI) with a compound of formula (VII) 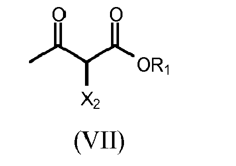 wherein:
R₁ is hydrogen, or a C₁-C₆ alkyl group optionally substituted with from 1 to 5 substituents selected from the group consisting of C₁-C₈ alkyl, halogen, and aryl
and
X₂ is a leaving group,
to give a compound of formula (VIII) 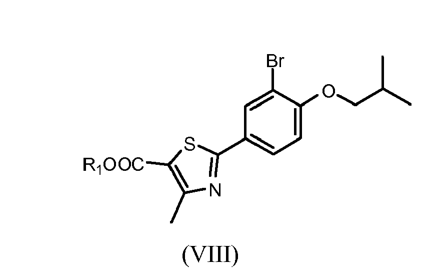 wherein R₁ is as defined above;
e) cyanating a compound of formula (VIII), as defined above, with potassium ferrocyanide, in the presence of a basic agent, and a catalytic system, wherein the catalytic system comprises a metal and optionally an organic ligand; to give the compound of formula (II) 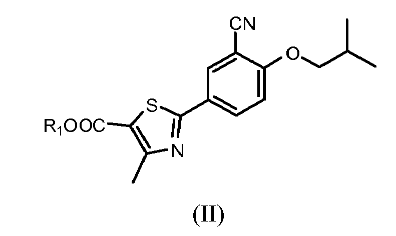 wherein R₁ is as defined above;
f) optionally hydrolysing the compound of formula (II), wherein R₁ is C₁-C₆ alkyl group optionally substituted with from 1 to 5 substituents selected from the group consisting of C₁-C₈ alkyl, halogen, and aryl.

2. The process according to claim 1, wherein R₁ is hydrogen, or ethyl.

3. The process according to claim 1, step c), wherein the solvent is a polar aprotic solvent.

4. The process according to claim 1, step c), wherein the molar ratio of sodium hydrosulphite with respect to substrate may be comprised from 1 to 5, preferably from 1 to 3.

5. The process according to claim 1, step c), wherein the magnesium chloride/sodium hydrosulphite ratio is comprised from 1:1 to 1:5, preferably from 1:1 to 1:2.

6. The process according to claim 1, step e), wherein the basic agent is sodium carbonate.

7. The process according to claim 1, step e), wherein the metal is a transition metal.

8. The process according to claim 7, wherein the transition metal is Pd or Cu.

9. The process according to claim 8, wherein the transition metal is Pd.

10. The process according to claim 1, step e), wherein the organic ligand may be selected from the group consisting of phosphine; trialkylphosphine; bidendate diphosphine ligand, comprising xanthene-based diphosphines; phenoxazine-based diphosphines; (oxydi-2,1-phenylene)bis(diphenylphosphine); 1,1'-bis(diphenylphosphino)ferrocene; or a bidentate amine ligand.
